# EUROPEAN PATENT APPLICATION

(11) **EP 4 454 633 A1**
(43) Date of publication of application: **30.10.2024**
(21) Application number: 23382383.0
(22) Date of filing: 24.04.2023
(51) Int. Cl.: A61K 8/02, A61K 8/06, A61K 8/34, A61Q 5/10

(54) **SOLID COLOURING AGENT EMULSION FOR MANUFACTURING HAIR DYE**

(71) Applicant: Roca Pares, Albert, 08037 Barcelona (ES)
(72) Inventor: Roca Pares, Albert, 08037 Barcelona (ES)
(74) Representative: Isern Patentes y Marcas S.L.

(57) **Abstract**

The invention relates to solid cosmetic compositions comprising a cosmetically acceptable vehicle, at least one colour precursor, at least one antioxidant agent, at least one preservative, at least one chelating agent, at least one treating agent and at least one antioxidant agent. The invention also relates to processes for preparing the solid cosmetic compositions and their use in preparing base creams for hair dyes.

## Description

### SECTOR OF THE TECHNIQUE

This invention refers to the field of dying keratin fibres and more specifically to the field of dying hair.

This invention refers to a solid colouring agent composition for producing colouring cream for hair dye. More specifically, the invention relates to solid cosmetic compositions comprising a cosmetically acceptable vehicle, at least one colour precursor, at least one antioxidant agent, at least one preservative, at least one chelating agent, at least one treating agent and at least one antioxidant agent.

### BACKGROUND OF THE INVENTION

Hair dyes are decorative cosmetics whose purpose is to change the colour or bleach the hair temporarily, semi-permanently or permanently, depending on their components (oxidative or not) and their penetration at different levels of the stem cortex. They are used by men and women to change the natural colour of the hair, delay the appearance of grey hair or re-pigment the hair when the grey is already established.

Hair dyes are classified based on their origin as: vegetable dyes, mineral or metallic dyes and synthetic dyes. The latter are classified according to the resistance of the colour to successively washing the hair and the time the colour remains on the hair. Thus, in the (i) Temporary dyes: The colour lasts a few days. High molecular weight molecules are used that remain on the surface of the cuticle. (ii) Semi-permanent dyes. They can be non-oxidative and oxidative, the colour lasts for weeks. Low molecular weight molecules are used and are introduced superficially into the cortex and (iii) Permanent dyes. The colour lasts indefinitely. Very low molecular weight molecules are used that go deep into the cortex. (Cosmetic Dermatology: Products and Procedures Edited by Zoe Diana Draelos © 2010 Blackwell Publishing Ltd. ISBN: 978-1-405-18635-3).

Two types of oxidation colouring can be distinguished: tone-on-tone oxidation colouring and permanent oxidation colouring. Tone-on-tone oxidation colouring: The hair is dyed in its natural colour or only modified by one tone (does not contain ammonia). Permanent oxidation colouring: It can eliminate melanin pigments (eumelanin pheomelanin). The hair is bleached and then dyed in lighter tones, in the same tone or darker. Depending on the oxidant concentration, a permanent oxidation colour can lighten from 1 to 4 tones (5-6 tones with ammonia load) while it can darken 7-8 tones in a controlled manner with a light oxidant load.

The active components of oxidation dyes are the colouring cream and the oxidant. Due to its chemical specifications, the colouring cream is sensitive to oxidation and therefore it is generally marketed in 30 to 100 ml aluminium tubes and the oxidising cream in plastic containers.

Seven main components with very specific functions can be found in most oxidative hair colouring creams that are currently produced:
(i) Base cream: In most cases, the base cream is an emulsion with an oil component and an aqueous component.
(ii) Colour precursors: Colour precursors are colourless molecules that can penetrate the hair to form large colour molecules.
(iii) Alkalising agents have a dual purpose: they open the cuticle layer of the hair so that the active ingredients, such as colour precursors, can penetrate the hair. The second purpose of the alkalising agent is to catalyse the oxidative reaction between colour precursors and hydrogen peroxide. Ammonia is the most common alkalising agent. Ammonia-free dyes work with monoethanolamine (MEA), a combination of two alkalising agents, for example, monoethanolamine and aminomethylpropanol (AMEA) or aminomethylpropanol (AMP).
(iv) Treating Agents: those that nourish the hair and at the same time protect the scalp and skin from the chemical action of the dye components.
(v) Antioxidants: help to prevent premature oxidation of colour precursors during storage of the colouring cream for hair dye.
(vi) Chelating agents: these ingredients "sequester" any metallic impurities that may be in the colouring cream to prevent an unwanted reaction with the hydrogen peroxide.
(vii) Perfume: The purpose of the perfume is to conceal the unpleasant smell of the ammonia.

Companies that produce hair dyes have additional problems to those of other companies in the cosmetics sector. Effectively, there is an average of 28 ingredients in a formula for a generic colouring cream that are distributed as detailed in Table 1 according to the aforementioned components. The base cream, the colour precursors and the treating agents are the main ingredients. The base cream represents approximately 87% by weight of the total formula.

**Table 1. Composition of a hair dye**

| **Hair dye** | **% by weight** | **Number of ingredients** |
|---|---|---|
| Base cream | 87.3 | 10 |
| Colour precursors | 2.6 | 5 |
| Alkalising ingredient | 8.0 | 1 |
| Chelating agent | 0.7 | 4 |
| Treating agent | 0.3 | 4 |
| Antioxidant/Preservative | 0.6 | 3 |
| Perfume | 0.6 | 1 |

A second aspect of vital importance that makes colouring cream production difficult is related to the state in which these 28 raw materials are marketed and their percentage in the final formula.

In preparing conventional hair dyes, more than 60% of the formula is made up of ingredients in a liquid state and the rest are solid. The fact that a significant part is solid poses mixing and homogenisation problems that lead to the use of a hot manufacturing process.

The components in which the proportion of ingredients in the final colouring cream formula is lower include the colour precursors and the so-called treating agents. Specifically, the colour precursors, which are the fundamental part of the final product, are in a solid state and in different formats (crystal or powder) and therefore to achieve a homogeneous formulation in which the percentage of the product's ingredients responds to the desired formula, it must be worked in batches by weighing the precursors depending on the colour to be manufactured.

It should be taken into account that in oxidation colouring cream compositions, the most commonly used colour precursors are oxidation bases such as p-phenylenediamine, p-toluenediamine, o-chloro-p-phenylenediamine, o-aminophenol and p-aminophenol and couplers such as m-phenylenediamine, 2,6-diaminotoluene, resorcinol and m-aminophenol. Colour precursors are generally compounds whose handling presents various hazards to human health. Therefore, in the process of weighing the ingredients, workers must comply with all workplace health and safety regulatory requirements in terms of hours of exposure to these products and the PPE to be used.

The batch manufacturing process for colouring creams for hair dyes is generally carried out in a multi-step process. First, all the ingredients are weighed according to the formula, then demineralised water is added, its temperature is raised and the waxes added, which melt on direct contact with hot demineralised water. Next, the rest of the hot demineralised water is added to the main mixing tank and the remaining previously-weighed raw materials are added manually. The batch is mixed until the ingredients are completely melted and then cooled, then alkalising agents and finally the perfume are added before packaging. This process can take between 1 and 6 hours depending on the batch volume.

Currently, some manufacturing companies in the world have developed manufacturing in three steps. These systems are made up of two premix tanks (wax tank and dye tank) and a main mixing tank where production is completed. In large facilities, the premixes prepared in the wax and stain premix tanks are transferred to the main mixing tanks using vacuum to avoid oxidation and possible contamination.

In the wax premix tank, solid waxes are added manually and melted inside the tank. The tank is continuously stirred until the waxes are completely melted, dispersed and dissolved. In the dye premix tank, the solid dyes are dissolved with hot water. The tank is continuously stirred until the dyes are fully dispersed and dissolved. According to the batch preparation sequence, the content of the tanks, waxes and dyes is transferred to the main mixing tank by vacuum. The batch temperature is raised to 80-85°C and the batch is recirculated through the homogeniser at the bottom. Subsequently, minor ingredients are added depending on the formula, followed by batch cooling. The ammonium hydroxide and perfume are then added slowly and the batch is finalised.

In any case, the colour precursor batch weighing process implies that the time spent working on it and therefore the minutes of exposure of the people who do so are strictly proportional to the number of kilograms manufactured per batch. The capacity of the manufacturing tank determines the batch volume. Therefore, if it is 100 kg, the batches will be 100 kg. The average weighing time for precursors, regardless of the volume to be produced and without taking the ingredient preparation time into account, is 10 minutes. Thus, to manufacture 500 kg of a single colour in batches of 100 kg, five weighings are required. In other words, 50 minutes in total.

With the current state of the art, it is extremely difficult and expensive to produce small amounts of colouring cream (for example, 10 kg or less for manufacturing very low-demand colours, samples or simply small productions) and that are exactly the same, since the colour precursor proportions (which are integrated in the solid state) are so low that a slight difference in weight has a very direct effect on the final result. It should be remembered that the weighing process is manual and, therefore, subject to human error. This point is especially critical in manufacturing an oxidation colouring cream, since the final result, or in other words, the colour that will finally result in human hair once applied, depends on the accuracy with which these ingredients are added, which individually usually represent less than 1% of the total formula.

Table 2 summarises this problem. To produce 500 kg of one colour dye, 13,000 g of five colour precursors (prec. 1 to prec. 5) need to be added that are distributed as detailed in the table in % and in grams. In order to manufacture 10 kg of the same colour, identical percentages are needed so that when weighing 500 kg, 28.08 g of precursor 1 must be added to the batch and 0.56 g to the 10 kg batch so that both productions contain 0.22% of this ingredient. For the precursor, 2,982.75 g and 19.65 g respectively, for the third quarter and fifth precursor 2,358.53 g, 2,817.13 g and 6,813.53 g in to produce 500 kg and theoretically 47.17 g, 56.34 g and 136.27 g to produce 10 kg.

In standard industrial colouring cream productions, +/- 0.25 g differentials can be admitted in adding one precursor or another without altering the colour of the resulting cream, since no alteration is observed to two decimal places in the formula percentage. It is observed that in producing 10 kg of dye (which is equivalent to about 200 50 ml tubes) the same absolute error of +/- 0.25 g per ingredient entails a significant change in the formula, since all the percentages vary and in the most extreme case, precursor 1, which should be present at 0.22% in the theoretical formula, becomes 0.31% in the product actually manufactured.

**Table 2. Simulation of the impact of a weighing error on the final product of +/- 0.25 g in the precursors depending on production volume.**

| | **Batch production** | | | |
|---|---|---|---|---|
| | **10 kg of colouring cream** | | **500 kg of colouring cream** | |
| | **Precursors (g)** | **% by weight** | **Precursors (g)** | **% by weight** |
| **Theoretical total** | 260.00 | 100.00 | 13000.00 | 100.00 |
| Precursor 1 | 0.56 | 0.22 | 28.08 | 0.22 |
| Precursor 2 | 19.65 | 7.56 | 982.72 | 7.56 |
| Precursor 3 | 47.17 | 18.14 | 2358.53 | 18.14 |
| Precursor 4 | 56.34 | 21.67 | 2817.13 | 21.67 |
| Precursor 5 | 1336.27 | 52.41 | 3813.53 | 52.41 |

| **SIMULATION ERROR EFFECT** | **Precursors (g)** | **% by weight** | **Precursors (g)** | **% by weight** |
|---|---|---|---|---|
| Real total | 259.75 | 100.00 | 12999.75 | 100.00 |
| Prec. 1 +0.25 g | 0.81 | 0.31 | 28.33 | 0.22 |
| Prec. 2 -0.25 g | 19.40 | 7.47 | 982.47 | 7.56 |
| Prec. 3 +0.25 g | 47.42 | 18.26 | 2358.78 | 18.14 |
| Prec. 4 -0.25 g | 56.09 | 21.59 | 2816.88 | 21.67 |
| Prec. 5 -0.25 g | 136.02 | 52.37 | 6813.28 | 52.41 |

Therefore, there is still a need for systems that allow large or very small productions without distinction that 100% guarantee the proportion of the different colour precursors in the final formula. Preferably, the system should enable hair dye to be manufactured simply and with minimal handling by operators and also enable weighing errors to be corrected during this process so that product batches with incorrectly weighed precursor amounts are not discarded or undesirable results reach the market.

There are also treating agents whose functions fundamentally act as hair conditioners (ease of combing, flexibility, softness and shine, volume and lightness) and at the same time act as an emollient with substances that soften the skin and create a protective lipid layer that prevents water loss through evaporation.

These conditioning and cosmetic functions of the treating agents are increasingly relevant given that permanent oxidation dyes have traditionally been formulated with ingredients that are aggressive to the hair and the skin.

As mentioned preciously, the batch manufacturing process for colouring creams for hair dyes is generally carried out in a multi-step process.

With the current state of the art, it is extremely difficult and costly to integrate all treating agents into a solid phase and to ensure that they are exactly the same as each other, since the treating agent proportions are particularly low in the hair dye formula. It should be remembered that the weighing process is manual and, therefore, subject to human error.

Therefore, there is still a need for processes that enable manufactures to guarantee 100% the proportion of the different treating agents in the final formula and that can be processed at low temperatures. The system should preferably enable hair dye to be manufactured simply and with minimum handling by the operators and at the same time enable weighing errors to be corrected.

This invention provides a solution to these problems in the hair dye industry.

### SUMMARY OF THE INVENTION

In a first aspect, the invention refers to a solid colouring agent composition for preparing colouring cream for hair dyes that comprises a cosmetically acceptable vehicle, at least one colour precursor, at least one antioxidant agent, at least one preservative, at least one chelating agent and at least one treating agent.

In a second aspect, the invention refers to a process for preparing a solid colouring agent composition for preparing a colouring cream for hair dye according to any of the preceding claims, comprising the following steps:
a) Add the cosmetically acceptable vehicle;
b) Add the antioxidant agents, chelating agents, colour precursors and preservative agents in solid state.
c) Homogenise the mixture from the previous step;
d) Add the antioxidant agents, chelating agents, colour precursors and preservative agents in non-solid state.
e) Stir the mixture obtained in the previous step at a speed of at least 90 RPM, preferably between 90 and 120 RPM;
f) Sieve the above mixture;
g) Pelletise the mixture resulting from step e)
h) Optionally, repeat step e) and f); and
i) Optionally, laminate the mixture resulting from step f) or step g); and
j) Package the product resulting from step f), g) or h).

In a third aspect, the invention refers to the use of the solid colouring agent composition of the first aspect of the invention or prepared using the process according to the second aspect of the invention for preparing colouring cream for hair dye or for dying hair.

### BRIEF DESCRIPTION OF THE DRAWINGS

Figure 1. - Comparison of the colouring results of several No. 6 dyes on the market (B, C and D) and the No. 6 dye made from the solid colouring agent composition of the invention (A).
Figure 2. - Colouring results for various colour ranges of dyes made from the solid colouring agent composition of the invention (A).

### PREFERRED EMBODIMENT OF THE INVENTION

In a first aspect, the invention refers to a solid colouring agent composition for preparing colouring cream for hair dye that comprises a cosmetically acceptable vehicle, at least one colour precursor, at least one antioxidant agent, at least one preservative, at least one chelating agent, at least one treating agent and at least one antioxidant agent. In a preferred embodiment of the first aspect of the invention, the solid colouring agent composition is a solid emulsion. In a preferred embodiment of the first aspect, the solid colouring agent emulsion is in the form of granules, flakes, powder or noodles, preferably in the form of granules. The granule format allows the best dosing precision and very fast dissolution and integration, which reduces production time and energy consumption.

The cosmetic terms to which reference is made have been defined according to the state of the art. Specifically, they have been defined according to the International Nomenclature for Cosmetic Ingredients, INCl.

The term "cosmetically acceptable vehicle" refers to a compound or a mixture of compounds in which the different compounds that comprise the concentrated cosmetic gel can dissolve and/or mix. The cosmetically acceptable vehicle can include solvents, surfactants, emulsifiers, anti-foaming agents and/or viscosity controllers.

The term "antioxidant agent" refers to a compound that inhibits reactions caused by oxygen, thus preventing oxidation and rancidity.

The term "preservative" refers to a compound that primarily inhibits microorganism growth in cosmetics.

The term "chelating agent" refers to a compound that reacts and forms complexes with metal ions that could affect the stability and/or appearance of cosmetics.

The term "colour precursor" refers to a compound that colours the cosmetic product and/or gives colour to the skin and/or its appendages, i.e. hair.

The term "treating agent" refers to a compound that nourishes the hair and protects it against the chemical action of the dye components. These act as hair conditioners (easy to comb, flexible, soft and shiny and/or give volume, lightness and shine) and at the same time act as an emollient with substances that soften the skin and create a protective lipid layer that prevents water loss due to evaporation.

The term "chemical dyes" refers to non-natural-origin compounds that colour the cosmetic product and/or give colour to the skin and/or its appendages.

The term "natural dyes" refers to compounds or extracts of natural origin that colour the cosmetic product and/or give colour to the skin and/or its appendages. Natural dyes can be vegetable origin or mineral origin.

The term "hair dye" refers to a compound or composition that colours hair.

The invention is briefly summarised and described in detail with reference to the following specifications and non-limiting examples. Unless otherwise specified, all percentages are percentages by weight.

The composition of the first aspect makes it possible to manufacture hair dye from 3 ingredients or raw materials instead the more than 20 that are currently used, which represents a significant change compared to the current state of the art.

In a preferred embodiment of the first aspect, the cosmetically acceptable vehicle comprises at least one of the compounds selected from water, isopropyl alcohol, propylene glycol, sodium lauryl sulphate, polyacrylic acid, sodium sulphate, coconut betaine, glycol stearate, polyethylene glycol monostearate (100), C12-C20 saturated-fatty-acid alcohols, ECOROL^{®} 68/50 or mixtures thereof. In a more preferred embodiment, the cosmetically acceptable vehicle consists of isopropyl alcohol, propylene glycol, sodium lauryl sulphate, polyacrylic acid, sodium sulphate, coconut betaine, glycol stearate and polyethylene glycol monostearate (100) and C12-C20 saturated-fatty-acid alcohols.

In another preferred embodiment of the first aspect, at least one colour precursor comprises at least one colour precursor selected from the group of chemical dyes, natural dyes or mixtures thereof.

In another preferred embodiment of the first aspect, at least one colour precursor comprises organic vegetable-origin natural dyes such as red henna, neutral henna, black henna, chamomile flower, sandalwood, black tea, *Rhamnus frangula* bark, sage, logwood, *Rubia tinctorum* root, acacia extract (catechu), cedar root or alkanna root.

In another preferred embodiment of the first aspect, at least one colour precursor comprises mineral-origin inorganic natural dyes, such as iron trioxide, titanium dioxide or mica.

In another preferred embodiment of the first aspect of the invention, at least one colour precursor is selected from the following group: 2,4-Diaminophenoxyethanol hydrochloride, 1,3-bis-(2,4-diaminophenoxy)-propane tetrahydrochloride, 2,6-diaminopyridine, 2-amino-3-hydroxypyridine, 3-methyl-4-aminophenol, 2-amino-4-hydroxyethylaminoanisole sulphate, 6-amino-m-cresol, 5-amino-6-chloro-o-cresol, 2-amino-6-chloro-4-nitrophenol, toluene-2,5-diamine sulphate, 4-chlororesorcinol, p-phenylenediamine, 1-Naphthol, N-phenyl-p-phenylenediamine, N-phenyl-p sulphate - phenylamine, 3-nitro-p-hydroxyethylaminophenol, 4-(2-hydroxyethylamino)-3-nitrotoluene, N,N-bis(2-hydroxyethyl)-p-phenylenediamine sulphate, 1-hydroxyethyl-4,5- Diaminopyrazole, 2-methylresorcinol, 4-amino-2-hydroxytoluene, 2-methyl-5-hydroxyethylaminophen, p-aminophenol, 4-methylaminophenol sulphate, phenylmethyl-pyrazolonaresorcinol, 4-hydroxypropylamino-3-nitrophenol, tetraaminopyrimidine sulphate, 2,6-dihydroxyethylaminotoluene, hydrochloride Hydroxyethyl-3-4-methylenedioxyaniline, 4-Amino-3-Nitrophenol, 2,4-diaminophenoxyethanol sulphate, HC blue 12, HC blue 2, HC blue 16, 2-Nitro-4'-hydroxydiphenylamine, HC red 1, Hydrochloride 2,2'-[(4-amino-3-nitrophenyl)imino]bisethanol, N1-(2-hydroxyethyl)-2-nitro-p-phenylenediamine, 2-(4-Amino-3-Nitroaniline) Ethanol, N,N '-Bis(2-Hydroxyethyl)-2-Nitro-p-Phenylenediamine, N-(2-hydroxyethyl)-2-nitro-4-trifluoromethylaniline, N-[(2-nitrophenyl)amino]ethanol, 2-[bis(2-hydroxyethyl)amino]-5-nitrophenol, N,N'-Bis(2-Hydroxyethyl)-2 Nitro-p-Phenylenediamine, HC blue 15, Basic Orange 31, Basic red 51, Basic violet 2 (CI 42520), Basic yellow 28, Basic yellow 51, Basic yellow 87, Sodium 4-amino-5-hydroxy-3-(4-nitrophenylazo)-6-(phenylazo)2,7-naphthalene-disulphonate (CI 20470), Acid blue 9, disodium 2,2'-(9,10-dioxoanthracene-1,4-diyldiimino)bis(5-methylsulphonate) (CI 61570), sodium 4-[(2-hydroxy-1-naphthylazo]benzenesulphonate (CI 15510), disodium 6-hydroxy-5-[(2-methoxy-4-sulphonato-m-tolyl)azo]naphthalene-2-sulphonate (CI 16035), (CI 42520), fluorescein-2,4,5,7-tetrabromo-12,13,14,15-tetrachloro disodium, Acid Yellow 23 (CI 60730), Acid Yellow 1 (CI 10316), Acid Yellow 1 (CI 19140), Acid Yellow 3 (CI 47005), Fluorescein Sodium (C! 45350), Hydroxyethyl-p-phenylenediamine sulphate, D-erythro-hex-3-enoic acid gamma-Lactone, Basic Blue 99, Basic Brown 16, Basic Orange 1, Basic Violet 16, CI Disperse Black 9, CI Disperse Violet 1 (CI 61100), Acid Red 33, Acid Violet 43 (CI 60730), 4-nitro-o-phenylenediamine, 2,4-diaminophenetol sulphate, 1-phenyl-3-methyl-5-pyrazolone, 8-hydroxyquinoline hemisulphate hemihydrate or mixtures thereof.

In another more preferable embodiment, the colour precursor comprises at least chemical dyes, such as 2,5-diaminotoluene sulphate, resorcinol, 4-aminophenol and its isomers, 4-amino-2-hydroxytoluene, 1-phenyl-3-methyl -5-pyrazolone, 2,4-diaminophenoxyethanol, 1-naphthol, 2,7-naphthalenediol, 2-amino-4-hydroxyethylamino anisole sulphate, 2-methylresorcinol, 4-amino-m-resorcinol, 4-chlororesorcinol, sulphate N,N-bis(2-hydroxyethyl)-p-phenylenediamine, p-phenyldiamine or 2,5-diaminotoluene sulphate.

In a more preferred embodiment the colour precursor is selected at least from 2,5-diaminotoluene sulphate, resorcinol, 4-aminophenol and its isomers, 4-amino-2-hydroxytoluene, 1-phenyl-3-methyl-5-pyrazolone, 2,4-diaminophenoxyethanol, 1-naphthol, 2,7-naphthalenediol, 2-amino-4-hydroxyethylamino anisole sulphate, 2-methylresorcinol, 4-amino-m-resorcinol, 4-chlororesorcinol, N,N- sulphate bis(2-hydroxyethyl)-p-phenylenediamine, p-phenyldiamine, 2,5-diaminotoluene sulphate, iron trioxide, titanium dioxide, red henna, neutral henna, black henna, chamomile flower, sandalwood, black tea, *Rhamnus frangula* bark, sage, logwood, *Rubia tinctorum* root, acacia (catechu) extract, cedar root, alkanna root, mica or mixtures thereof.

In another preferred embodiment of the first aspect, at least one treating agent is selected from the group of Polyquaternium-6, Polyquaternium-22, dimethicone or aminopropyl phenyl trimethicone, hydrolysed keratin, high-erucate oil, *Argania spinosa* seed oil, *Camellia oleifera* seed oil, *European olive* fruit oil, *Prunus amygdalus dulcis* oil, Zea *mays* oil, *Persea gratissima* oil, *Vitis vinifera* seed oil, hydrolysed jojoba esters, *Rosmarinus officinalis* extract, honey extract or mixtures thereof.

In another preferred embodiment of the first aspect, at least one antioxidant agent is selected from ascorbic acid, sodium dithionate, sodium sulphanilate, thiolactic acid, erythorbic acid or mixtures thereof. In a more preferred embodiment, the antioxidant agent consists of ascorbic acid salts, sodium dithionate, sodium sulphanilate, thiolactic acid, erythorbic acid or mixtures thereof.

In another preferred embodiment of the first aspect, at least one chelating agent is selected from ethylenediaminetetraacetic acid (EDTA) or its salts, citric acid or its salts, tartaric acid or its salts, ethanolamine, sodium sulphate, etidronic acid, pentasodium pentetate, sodium silicate, sodium metasilicate, linoleamidopropyl PG-dimethicone phosphate dimonium chloride or mixtures thereof. In a more preferred embodiment, the chelating agent is ethylenediaminetetraacetic acid (EDTA) or its salts, preferably the sodium salt of EDTA.

In another preferred embodiment of the first aspect, at least one preservative agent is selected from 2-phenoxyethanol, hydroxyethylcellulose, tocopherol, sodium metabisulphite, steartrimonium chloride, behentrimonium chloride or mixtures thereof. In a more preferred embodiment, the preservative agent consists of 2-phenoxyethanol, hydroxyethylcellulose, tocopherol, sodium metabisulphite, steartrimonium chloride, behentrimonium chloride or mixtures thereof.

In another preferred embodiment, the solid colouring agent cosmetic composition for preparing colouring cream for hair dye of the first aspect comprises:
- A cosmetically acceptable vehicle between 75% and 95% by weight with respect to the solid colouring agent cosmetic composition;
- At least one chelating agent between 0.5% and 4% by weight of the solid colouring agent cosmetic composition;
- At least one colour precursor between 0.1% and 10% by weight with respect to the solid colouring agent cosmetic composition;
- At least one treating agent between 0.2% 3% by weight of the solid colouring agent cosmetic composition;
- At least one preservative agent between 0.5% and 5% by weight of the solid colouring agent cosmetic composition;
- At least one antioxidant agent between 0.5% and 3% by weight with respect to the solid colouring agent cosmetic composition.

In another preferred embodiment, the solid colouring agent cosmetic composition for preparing colouring cream for hair dye of the first aspect comprises:
- A cosmetically acceptable vehicle between 80% and 90% by weight with respect to the solid colouring agent cosmetic composition;
- At least one chelating agent between 1.5% and 3% by weight with respect to the solid colouring agent cosmetic composition.
- At least one colour precursor between 0.1 % and 8% by weight with respect to the solid colouring agent cosmetic composition;
- At least one treating agent between 0.5% 1.5% by weight of the solid colouring agent cosmetic composition; and
- At least one antioxidant agent between 1.5% and 4% by weight with respect to the solid colouring agent cosmetic composition.
- At least one preservative agent between 1% and 3% by weight with respect to the solid colouring agent cosmetic composition;

In an even more preferred embodiment, the solid colouring agent cosmetic composition for preparing colouring cream for hair dye in the first aspect comprises:
- a mixture of water isopropyl alcohol, propylene glycol, sodium lauryl sulphate, polyacrylic acid, sodium sulphate, coconut betaine, glycol stearate and polyethylene glycol monostearate (100) and C12-C20 saturated-fatty-acid alcohols, between 80% and 90% by weight with respect to the solid colouring agent cosmetic composition;
- At least one chelating agent between 1.5% and 3% by weight of the solid colouring agent cosmetic composition; and
- At least one colour precursor between 0.1% and 8% by weight with respect to the solid colouring agent cosmetic composition;
- At least one antioxidant agent between 0.5 and 1.5% by weight with respect to
   the solid colouring agent cosmetic composition;
- At least one preservative agent between 1.5% and 4% by weight of the solid colouring agent cosmetic composition;
- At least one treating agent between 1% and 3% by weight with respect to the solid colouring agent cosmetic composition.

In another preferred embodiment, the solid colouring agent emulsion for preparing colouring cream for hair dye of the first aspect comprises:
- A cosmetically acceptable vehicle between 75% and 95% by weight with respect to the solid colouring agent emulsion;
- At least one chelating agent between 0.5% and 4% by weight of the solid colouring agent emulsion;
- At least one colour precursor between 0.1% and 10% by weight with respect to the solid colouring agent emulsion;
- At least one treating agent between 0.2% and 3% by weight of the solid colouring agent emulsion;
- At least one preservative agent between 0.5% and 5% by weight of the solid colouring agent emulsion;
- At least one antioxidant agent between 0.5% and 3% by weight with respect to the solid colouring agent emulsion.

In another preferred embodiment, the solid colouring agent emulsion for preparing colouring cream for hair dye of the first aspect comprises:
- A cosmetically acceptable vehicle between 80% and 90% by weight with respect to the solid colouring agent emulsion;
- At least one chelating agent between 1.5% and 3% by weight with respect to the solid colouring agent emulsion;
- At least one colour precursor between 0.1 % and 8% by weight with respect to the solid colouring agent emulsion;
- At least one treating agent between 0.5% and 1.5% by weight of the solid
   colouring agent emulsion;
- At least one antioxidant agent between 1.5% and 4% by weight with respect to the solid colouring agent emulsion.
- At least one preservative agent between 1% and 3% by weight with respect to the solid colouring agent emulsion;

In an even more preferred embodiment, the solid colouring agent emulsion for preparing colouring cream for hair dye of the first aspect comprises:
- A mixture of water, isopropyl alcohol, propylene glycol, sodium lauryl sulphate, polyacrylic acid, sodium sulphate, coco betaine,
   glycol stearate and polyethylene glycol monostearate (100) C12-C20 saturated fatty-acid alcohols, between 80% and 90% by weight with respect to the solid colouring agent emulsion;
- At least one chelating agent between 1.5% and 3% by weight of the solid colouring agent emulsion; and
- At least one colour precursor between 0.1 % and 8% by weight with respect to the solid colouring agent emulsion;
- At least one antioxidant agent between 0.5 and 1.5% by weight with respect to the solid colouring agent emulsion;
- At least one preservative agent between 1.5% and 4% by weight with respect to
the solid colouring agent emulsion;
- At least one treating agent between 1% and 3% by weight with respect to the solid colouring agent emulsion.

In a preferred embodiment, the solid colouring agent cosmetic emulsion comprises less than 3% water with respect to the solid colouring agent cosmetic emulsion, preferably less than 2% by weight of water with respect to the solid colouring agent cosmetic emulsion.

In a second aspect, the invention refers to a process for preparing a solid colouring agent emulsion for preparing a colouring cream for hair dye according to any of the preceding claims, comprising the following steps:
a) Add the cosmetically acceptable vehicle;
b) Add the antioxidant agents, chelating agents,
   colour precursors and preservatives in solid state,
c) Homogenise the mixture from the previous step;
d) Add the antioxidant agents, chelating agents, colour precursors and preservative agents in non-solid state,
e) Homogenise the mixture from the previous step;
f) Stir the mixture obtained in the previous step at a speed of at least 90 RPM, preferably between 90 and 120 RPM;
g) Sieve the above mixture;
h) Pelletise the mixture resulting from step e)
i) Optionally, repeat step e) and f); and
j) Optionally, laminate the mixture resulting from step f) or step g); and
k) Package the product resulting from step f), g) or h).

In a preferred embodiment of the second aspect of the invention, steps a), b), c) and d) are carried out at less than 50°C.

In an even more preferred embodiment of the second aspect of the invention, the process for preparing a solid colouring agent emulsion for preparing colouring cream for hair dye according to any of the preceding claims comprises the following steps:
a) Add the cosmetically acceptable vehicle;
b) Add the antioxidant agents, chelating agents, colour precursors and preservative agents in solid state,
c) Homogenise the mixture from the previous step at less than 50°C;
d) Add the antioxidant agents, chelating agents, colour precursors and preservative agents in a non-solid state,
e) Homogenise the mixture from the previous step at less than 50°C;
f) Stir the mixture obtained in the previous step at a speed of at least 90 RPM, preferably between 90 and 120 RPM;
g) Sieve from the previous mixture;
h) Pelletise the mixture resulting from step e)
i) Optionally, repeat step e) and f); and
j) Optionally, laminate the mixture resulting from step f) or step g); and
k) Package the product resulting from step f), g) or h).

The third aspect of the invention refers to the use of the solid colouring agent composition according to the first aspect of the invention or prepared using the process according to the second aspect of the invention in preparing colouring cream for hair dye or in preparing the hair dye.

### Examples

Specific and preferred modalities of the solid colouring agent composition and the preparation process thereof have been previously described without prejudice to the fact that changes contemplated by an expert person on the matter can be made without departing from the scope of this invention defined in the claims that follow.

### Example 1: Formulation of a solid colouring agent cosmetic composition according to the invention.

In this particular example, the solid cosmetic composition comprises various treating agents such as *Argania spinosa* seed oil or *Persea gratissima* oil. Final amounts may vary depending on the type and number of treating agents in the final formula.

**Table 2. Formulation example of the solid cosmetic composition of the invention.**

| **Component** | **CAS number** | **% by weight** |
|---|---|---|
| **Cosmetically acceptable vehicle** | - | **85.06%** |
| Water | 7732-18-5 | 1.08% |
| Behentrimonium chloride | 17301-53-0 | 0.56% |
| Ceteth-24 | 9004-95-9 | 7.86% |
| Cetearyl alcohol | 67762-27-0 | 54.63% |
| Isopropyl alcohol | 67-63-0 | 3.60% |
| Isopropyl myristate | 110-80-1 | 0.37% |
| Oleic acid | 112-80-1 | 0.56% |
| Oleyl alcohol | 143-28-2 | 0.56% |
| Propylene glycol | 57-55-6 | 14.41% |
| Sodium laureth sulphate | 3088-31-1 | 1.44% |
| **Treating agent** | - | 0.93% |
| Argania spinosa seed oil | 299184-75-1 | 0.19% |
| Oleyl erucate / (Z)-octadec-9-enyl (Z)-docos-13-enoate | 17673-56-2 | 0.19% |
| Persea gratissima oil | 8024-32-6 | 0.19% |
| Prunus amygdalus dulcis oil | 8007-69-0 | 0.37% |
| **Antioxidant agents** / **Preservatives** | - | **3.44%** |
| Hydroxyethylcellulose | 9004-62-01 | 0.54% |
| Phenoxyethanol | 122-99-6 | 1.44% |
| Sodium ascorbate | 134-03-2 | 1.46% |
| **Chelating agents** | - | **2.61%** |
| Behentrimonium chloride | 17301-53-0 | 1.08% |
| Sodium lauroyl glutamate | 29923-31-7 | 0.45% |
| Tetrasodium EDTA | 64-02-8 | 1.08% |
| **Colour precursor** | - | **7.96%** |
| 2,4-diaminophenoxyethanol HCl | 66422-95-5 | 0.02% |
| 4-amino-hydroxytoluene | 2835-95-2 | 0.26% |
| m-Aminophenol | 591-27-5 | 0.57% |
| p-Aminophenol | 123-30-8 | 1.36% |
| Phenyl methyl pyrazolone | 89-25-8 | 0.20% |
| Resorcinol | 108-46-3 | 1.62% |
| 2.5-diaminotoluene sulphate | 615-50-9 | 3.93% |

### Example 2: Preparing the solid colouring agent cosmetic composition of the invention

The solid colouring agent cosmetic composition of Example 1 was prepared following these steps:
a) Add the cosmetically acceptable vehicle;
b) Add the antioxidant agents, chelating agents, colour precursors and preservative agents in solid state
c) Homogenise the mixture from the previous step at less than 50°C;
d) Add the antioxidant agents, chelating agents, colour precursors and preservative agents in the non-solid state
e) Homogenise the mixture from the previous step at less than 50°C;
f) Stir the mixture obtained in the previous step at a speed of at least 110 RPM;
g) Sieve the above mixture;
h) Pelletise the mixture resulting from step e);
i) Optionally, repeat step e) and f); and
j) Optionally, laminate the mixture resulting from step f) or step g); and
k) Package the product resulting from step f), g) or h).

The solid colouring agent cosmetic composition is stable for at least 36 months and once the container of the solid cosmetic composition is opened it is stable for 8 weeks.

### Example 3: Preparing hair dyes by means of the solid colouring agent cosmetic composition of the invention.

The solid colouring agent cosmetic composition of Example 1 was used to prepare the following hair dye with ammonia and without ammonia. The compounds and amounts of colour precursor are adjusted depending on the desired tone. In this particular example, the solid colouring agent cosmetic composition in Table 3 would correspond to a Number 6 tone, which is a tone that exists in all the colour charts of practically all hair dye manufacturers in the world and which means the results can be compared with a dye manufactured from the solid colouring agent cosmetic composition.

**Table 3. Example of hair dye prepared with the concentrated cosmetic composition from Example 1 without ammonia.**

| **Component** | **% by weight** |
|---|---|
| Water | 65.4 |
| Solid colouring agent emulsion | 28.0 |
| Monoethanolamine | 6.0 |
| Perfume - Champion^{®} | 0.6 |

The production process of the base cream for hair dye consists of the following steps:
a) Add the deionised water
b) Add the solid colouring agent emulsion
c) Homogenise the mixture at 85-90°C; and
d) Emulsify by stirring between 90 and 120 RPM;

The base cream preparation process enables weights to be corrected by adding only two elements in its preparation, reducing the need for specialist operators, the preparation time, handling errors and the risks to operators.

To prepare the hair dye from the base cream obtained through the above process, proceed as follows:
a) Add the alkalising agent at 60-75°C;
b) Cool to less than 50°C
c) Add perfume;
d) Cool the mixture obtained in the previous step; and
e) Package the resulting hair dye.

This process enables colouring cream for hair dye to be prepared in the simplest and fastest way possible, avoiding handling colour precursors, as they are already incorporated into the solid cosmetic emulsion, and minimising errors in weighing the minor ingredients of the traditional formula, as these are already incorporated and homogenised in a solid colouring agent composition.

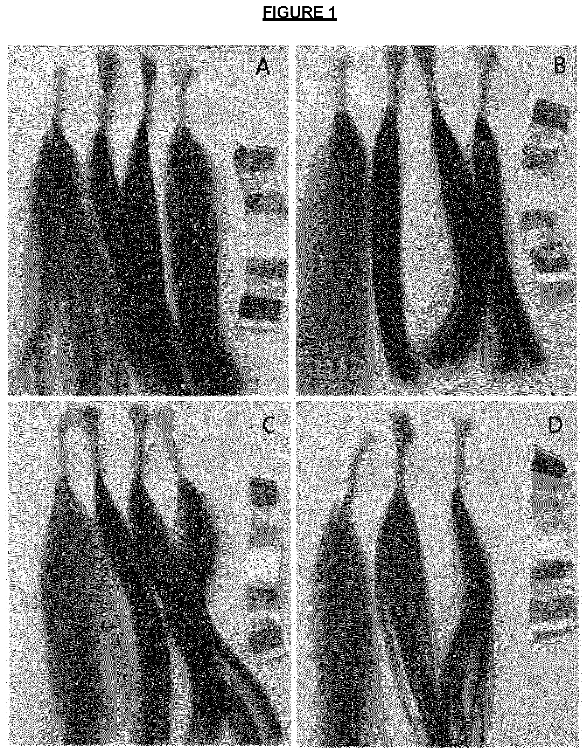

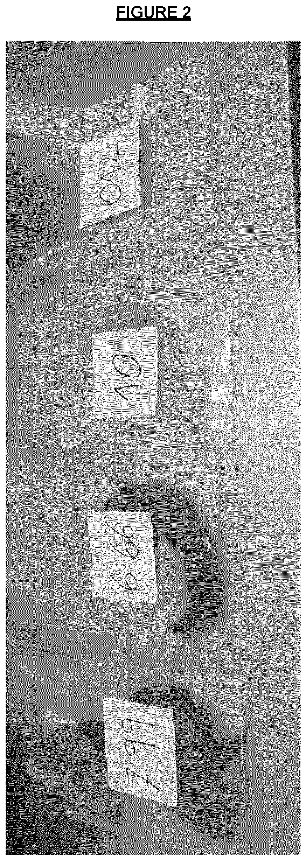

The colouring obtained with a hair dye made from the solid colouring agent emulsion of the invention is comparable to the colouring of any dye prepared by conventional state-of-the-art processes as seen in the images in figure 1 and figure 2.

## Claims

1. A solid cosmetic colouring agent composition for preparing colouring cream for hair dye comprising a cosmetically acceptable vehicle, at least one treating agent, at least one colour precursor, at least one chelating agent, at least one preservative and at least one antioxidant agent.

2. The solid cosmetic colouring agent composition according to the preceding claim, wherein the solid cosmetic colouring agent composition is a solid emulsion.

3. The solid cosmetic colouring agent composition according to any preceding claim, wherein the composition is in the form of granules, flakes, powder or noodles, preferably in the form of granules.

4. The solid cosmetic colouring agent composition according to any preceding claim, comprising:
- A cosmetically acceptable vehicle between 75% and 95% by weight with respect to the solid cosmetic composition;
- At least one chelating agent between 0.5% and 4% by weight with respect to the solid cosmetic composition;
- At least one colour precursor between 0.1% and 10% by weight with respect to the solid cosmetic composition;
- At least one treating agent between 0.2% 3% by weight of the solid cosmetic composition; and
- At least one antioxidant agent between 0.5% and 5% by weight with respect to the solid cosmetic composition.
- At least one preservative agent between 0.5% and 3% by weight with respect to the solid cosmetic composition.

5. The solid cosmetic colouring agent composition according to any preceding claim, comprising:
- A cosmetically acceptable vehicle between 80% and 90% by weight with respect to the solid cosmetic composition;
- At least one chelating agent between 1.5% and 3% by weight with respect to the
solid cosmetic composition;
- At least one colour precursor between 0.1% and 8% by weight with respect to the solid cosmetic composition;
- At least one treating agent between 0.5% 1.5% by weight of the solid cosmetic composition; and
- At least one antioxidant agent between 1.5% and 4% by weight with respect to the solid cosmetic emulsion.
- At least one preservative agent between 1% and 3% by weight with respect to the solid cosmetic composition.

6. The solid cosmetic colouring agent composition according to any preceding claim, wherein the vehicle cosmetically comprises behentrimonium chloride, cetearyl alcohol, polyethylene glycol (24) cetyl ether (Ceteth-24), isopropyl myristate, oleic acid and oleyl alcohol, preferably consisting of behentrimonium, cetearyl alcohol, polyethylene glycol(24) cetyl ether (Ceteth-24), isopropyl myristate, oleic acid and oleyl alcohol.

7. The solid cosmetic colouring agent composition according to any preceding claim, where the treating agent is selected from high-erucate oil, *Argania spinosa* seed oil, *Camellia oleifera* seed oil, European olive fruit oil, *Prunus amygdalus dulcis* oil, Zea *mays* oil, *Persea gratissima* oil, *Vitis vinifera* seed oil, hydrolysed jojoba esters, *Rosmarinus officinalis* extract, honey extract or mixtures thereof.

8. The solid cosmetic colouring agent composition according to any preceding claim, wherein at least one colour precursor is selected from the group of chemical dyes, natural dyes or mixtures thereof.

9. The solid cosmetic colouring agent composition according to any preceding claim, wherein at least the colour precursor is selected from 2,5-diaminotoluene sulphate, resorcinol, 4-aminophenol and its isomers, 4-amino-2-hydroxytoluene, 1-phenyl-3-methyl-5-pyrazolone, 2,4-diaminophenoxyethanol, 1-naphthol, 2,7-naphthalenediol, 2-amino-4-hydroxyethylamino anisole sulphate, 2-methylresorcinol, 4-amino-m-resorcinol, 4-chlororesorcinol, sulphate N,N-bis(2-hydroxyethyl)-p-phenylenediamine 35, p-phenyldiamine, 2,5-diaminotoluene sulphate, iron trioxide, titanium dioxide, red henna, neutral henna, black henna, chamomile flower, sandalwood, black tea, *Rhamnus frangula* bark, sage, logwood, *Rubia tinctorum* root, acacia (catechu) extract, cedar root, alkanna root, mica or mixtures thereof.

10. The solid cosmetic colouring agent composition according to any preceding claim, wherein at least one antioxidant agent is selected from ascorbic acid salts, sodium dithionate, sodium sulphanilate, thiolactic acid, erythorbic acid or mixtures thereof.

11. The solid colouring agent cosmetic composition according to any preceding claim, wherein at least one preservative agent is selected from 2-phenoxyethanol, hydroxyethylcellulose, tocopherol, sodium metabisulphite, steartrimonium chloride, behentrimonium chloride or mixtures thereof.

12. The solid cosmetic colouring agent composition according to any preceding claim, where at least one chelating agent is selected from ethylenediaminetetraacetic acid (EDTA) or its salts, citric acid or its salts, tartaric acid or its salts, ethanolamine, sodium sulphate, etidronic acid, pentasodium pentetate, sodium silicate, sodium metasilicate, linoleamidopropyl PG-dimonium chloride dimethicone phosphate or mixtures thereof.

13. A process for preparing a solid colouring agent cosmetic composition for preparing colouring cream for hair dye according to any of the preceding claims comprising the following steps:
a) Add the cosmetically acceptable vehicle;
b) Add the antioxidant agents, chelating agents, colour precursors and preservative agents in solid state;
c) Homogenise the mixture from the previous step;
d) Add the antioxidant agents, chelating agents,
colour precursors and preservative agents in non-solid state;
e) Homogenise the mixture from the previous step;
f) Stir the mixture obtained in the previous step between 90 and 120 RPM;
g) Sieve the above mixture;
h) Pelletise the mixture resulting from step e);
i) Optionally, repeat step e) and f);
j) Optionally, laminate the mixture resulting from step f) or step g); and
k) Package the product resulting from step f), g) or h).

14. The process for preparing the solid cosmetic composition for preparing a hair dye according to the previous claim, wherein steps a), b) c) and d) are carried out at less than 50°C.

15. Use of the solid colouring agent cosmetic composition according to any of the claims 1-9 or prepared by means of the process according to any of the claims 10-11 for preparing a base cream for hair dye or for preparing hair dye.
